# FASCICULE DE BREVET EUROPEEN

(11) **EP 0 885 568 B1**
(45) Date de publication et mention de la délivrance du brevet: **13.11.2002**
(21) Numéro de dépôt: 98401438.1
(22) Date de dépôt: 12.06.1998
(51) Int. Cl.: A23L 1/304, A23G 3/00, A61K 33/06

(54) **"Pâte gélifiée sans sucre à haute teneur en calcium"**
Kalziumreiche gelierte Paste ohne Zucker
Sugarless calcium rich gelled paste

(30) Priorité: 13.06.1997 FR 9707376
(43) Date de publication de la demande: 23.12.1998
(73) Titulaire: Diepharmex, 1201 Genève (CH)
(72) Inventeur: Nouvel-Rousselot, Colette, 75017 Paris (FR); Sancy, Yolande, 30000 Nimes (FR); Mortara, Ricardo, 1201 Genève (CH)
(74) Mandataire: Boulinguiez, Didier

(56) Documents cités:
- EP-A- 0 166 440
- EP-A- 0 346 866
- DE-U- 29 516 292
- FR-A- 2 000 278
- FR-A- 2 687 309
- US-A- 4 582 709
- DATABASE WPI Section Ch, Week 7850 Derwent Publications Ltd., London, GB; Class D13, AN 78-90419A XP002054601 & JP 53 127856 A (KAJI H), 8 novembre 1978

## Description

L'invention a pour objet une pâte gélifiée sans sucre à haute teneur en calcium.

Elle a également pour objet son procédé de préparation, ainsi que son utilisation dans le domaine diététique ou pharmaceutique, en vue notamment de pallier certaines déficiences et carences et de prévenir et combattre, entre autres, l'ostéoporose.

L'ostéoporose est une affection qui fragilise les os et entraîne des fractures. Elle se rencontre essentiellement chez les femmes, surtout après la ménopause, du fait de l'arrêt des sécrétions hormonales qui entraîne une réduction de la masse osseuse et du constituant minéral de l'os, qui se fragilise. Bien que touchant essentiellement les femmes, cette maladie n'épargne cependant pas les hommes, chez qui l'on observe 30 % des fractures du col du fémur et 20 % des tassements vertébraux. L'ostéoporose peut également apparaître comme la manifestation secondaire d'autres maux : certaines insuffisances hormonales (hypogonadisme), certaines prises médicamenteuses (en particulier les corticoïdes), les intoxications par l'alcool ou le tabac, certaines maladies hépatodigestives chroniques, sont ainsi des facteurs déclenchants ou aggravants.

Par ailleurs, certains comportements alimentaires rencontrés à l'heure actuelle chez de nombreux adolescents, surtout de sexe féminin, risquent d'aggraver ou de précipiter le phénomène. La mode des mannequins très minces et filiformes incite en effet beaucoup d'adolescentes à suivre des régimes alimentaires très stricts et très déséquilibrés, conduisant à l'apparition de déficiences en divers éléments, dont notamment le calcium.

Les apports en calcium-élément recommandés chez l'adulte sont estimés à 800-900 mg par jour. Ils servent à entretenir la minéralisation ou à limiter les pertes calciques. Or, les études publiées à ce jour montrent qu'en fait beaucoup d'adultes ont une consommation inférieure à ces préconisations. En effet, la consommation de très peu de lait (essentiellement sous forme de préparations culinaires), d'un morceau de fromage et d'un yaourt représente seulement un apport moyen quotidien de 300 à 400 mg, inférieur donc de moitié aux 800-900 mg recommandés.

Chez l'enfant et l'adolescent, ainsi que chez la femme lors des périodes de grossesse et d'allaitement, les besoins calciques sont encore plus importants et les apports recommandés sont de l'ordre de 1200 à 1500 mg par jour.

L'absorption d'un supplément calcique oral, de façon régulière, permettrait d'assurer ces besoins importants en calcium et permettrait de compenser les déficiences alimentaires constatées de plus en plus fréquemment chez les enfants et les adolescents. Elle pourrait aussi ralentir la détérioration osseuse et prévenir les risques d'ostéoporose et les troubles qui en découlent.

Il existe déjà de nombreux produits diététiques ou des formes pharmaceutiques orales contenant du calcium comme principe actif essentiel. La demande de brevet japonais n° 53-127856, par exemple, décrit ainsi des confiseries supplémentées en calcium obtenues à partir d'un mélange formé de 10 parties d'un sel de calcium d'acide organique alimentaire, 70 parties de sirop de glucose et 20 parties de saccharose, ledit mélange étant fondu, injecté et mis dans des moules convenables, fournissant au refroidissement les confiseries supplémentées en calcium. Ces préparations diététiques ou pharmaceutiques destinées à être administrées par voie buccale contiennent généralement jusqu'à 500 mg de calcium élémentaire par unité de prise, administré sous différents sels : carbonate, gluconate, phosphate notamment, et se présentent le plus souvent sous la forme de comprimés ou de sachets de poudre effervescente. La majorité des préparations pharmaceutiques contiennent en outre une faible quantité de vitamine D, connue pour augmenter l'absorption digestive du calcium.

Malheureusement, il s'avère que les produits diététiques ou les produits pharmaceutiques à haute teneur en calcium qui sont présents sur le marché ne sont pas toujours suffisamment attractifs, du point de vue de la forme, de la texture ou du goût, pour qu'ils puissent être consommés de manière continue et régulière, notamment par les enfants et les adolescents.

La Société Demanderesse s'est donc attachée à résoudre ce problème, en essayant de définir et de mettre au point une forme de produit diététique ou pharmaceutique à haute teneur en calcium qui puisse être consommée par tous les sujets ou patients, quel que soit leur âge.

Elle a eu le mérite de mettre au point, après de nombreux essais, une telle forme qui, grâce à sa présentation -pouvant éventuellement être ludique- à sa texture -pouvant convenir à toutes les personnes- et à son aromatisation et sa coloration -susceptibles d'être adaptées facilement aux diverses tranches d'âge- assure une prise régulière et continue par toutes les personnes, y compris les enfants et les adolescents.

Le produit diététique ou pharmaceutique à haute teneur en calcium conforme à l'invention est caractérisé par le fait qu'il se présente sous la forme d'une pâte gélifiée sans sucre et qu'il contient de 100 à 500 mg de calcium par article unitaire.

Au sens de la présente invention, on entend par le terme de "pâte gélifiée" les articles légers et aérés, à croquer ou à mâcher, qui sont généralement vendus sous les noms de "guimauves" ou "marshmallows" ou sous des noms spéciaux comme "têtes de nègre", "soufflage"... Ces produits, traditionnellement formulés à base de sucre (essentiellement saccharose et sirops de glucose) sont aérés en présence d'agents gélifiants tels que la gélatine, l'albumine d'oeuf, les pectines, la gomme arabique, l'agaragar, ou les produits connus sous la marque HYFOAMA, puis gélifiés après coulage à l'amidon, ou extrusion, ou dépôt sur table froide et coupage.

Toujours au sens de la présente invention, le terme "sans sucre" signifie que les produits selon l'invention ne comportent pas de sucres tels que notamment le saccharose, le glucose ou le fructose, ces sucres traditionnels ayant été remplacés par des produits comme les sucres-alcools, communément dénommés polyols, au rang desquels on peut citer notamment le sorbitol, le mannitol, le maltitol, le xylitol, l'érythritol et l'isomalt, ou par des produits peu caloriques tels que les polyglucoses ou les polyfructoses.

Les pâtes gélifiées sans sucre conformes à l'invention se présentent sous la forme d'articles unitaires d'un poids compris entre 0.5 g et 6 g, de préférence entre 1.5 g et 5.5 g, et plus préférentiellement encore entre 1.8 g et 5.2 g.

Selon une caractéristique préférentielle de l'invention, les produits destinés aux enfants se présentent sous la forme de pâtes d'un poids unitaire d'environ 2 à 3 g, tandis que les produits destinés aux adolescents et aux adultes se présentent sous la forme de pâtes d'un poids unitaire plus élevé, généralement compris entre 3 et 5 g.

Selon une autre caractéristique préférentielle de l'invention, les produits destinés aux enfants présentent une teneur en calcium-élément de 50 mg à 250 mg, de préférence de 100 mg à 250 mg et plus préférentiellement encore de 200 mg par article unitaire, alors que les produits destinés aux adultes et aux adolescents ont une teneur en calcium-élément de 250 mg à 1000 mg, de préférence de 250 mg à 500 mg et plus préférentiellement encore de 400 mg par article unitaire.

Les produits contenant une association calcium-vitamine D, essentiellement destinés à prévenir et combattre l'ostéoporose, sont de préférence présentés sous la forme d'articles unitaires d'un poids compris entre 3 et 5 g et contiennent, outre la vitamine D, de 200 à à 500 mg, de préférence 400 mg de calcium-élément.

Etant donné que le calcium est amené sous la forme de sels dans lesquels cet élément représente au plus 40 % en poids, il faut incorporer dans les pâtes gélifiées selon l'invention un pourcentage de sel de calcium qui est au minimum d'environ 10 % en poids par rapport au poids de l'article de pâte gélifiée, et qui peut aller jusqu'à environ 35 % en poids. Le plus souvent, ce pourcentage de sel de calcium est situé entre environ 15 et environ 25 % en poids. Ainsi, en prenant pour exemple un produit pour enfants d'un poids unitaire de 2400 mg, dosé à 200 mg de calcium-élément apporté sous forme de carbonate de calcium, le pourcentage de sel à incorporer dans la formulation est de 20.8 % environ.

Il est compréhensible que l'homme du métier, confronté à ce problème d'arriver à incorporer dans une forme galénique une quantité aussi élevée de sel de calcium, se soit tourné tout naturellement vers la réalisation de formes sèches, c'est-à-dire des comprimés ou des mélanges de poudres présentés sous forme de sachets. En aucun cas en effet, il ne pouvait logiquement imaginer pouvoir incorporer facilement un pourcentage d'environ 20 % de sel de calcium dans une pâte gélifiée du type guimauve ou marshmallow, article éminemment caractérisé par une texture aérée, souple et légère.

Et même si, par extraordinaire, l'homme du métier avait quand même considéré qu'il pouvait être possible d'incorporer un si haut pourcentage de sel de calcium dans la formulation d'une pâte gélifiée et de parvenir à réaliser une gélification satisfaisante du produit, il devait logiquement s'attendre à l'obtention d'un produit présentant des propriétés texturales et organoleptiques pour le moins déficientes, voire catastrophiques.

Or, la Société Demanderesse a réussi à surmonter ces préjugés très importants existant à l'encontre de la réalisation d'une pâte gélifiée contenant une très haute teneur en calcium -préjugés encore aggravés par le fait qu'il s'agissait également de réaliser une pâte gélifiée "sans sucre"- et elle a eu le mérite de mettre au point un tel produit, qui non seulement présente des caractéristiques texturales satisfaisantes, mais qui également et surtout possède une apparence et des qualités organoleptiques très attractives.

Conformément à l'invention, on utilise comme agents sucrants ou édulcorants des polyols, ou des produits de ballast faiblement caloriques tels que les polyglucoses ou les polyfructoses. Les polyols sont choisis parmi le sorbitol, le maltitol, le mannitol, l'érythritol, le xylitol, l'isomalt, les sirops de glucose hydrogénés, les sirops de sorbitol ou les sirops de maltitol. De préférence, on utilise le sorbitol, le maltitol, ainsi que des sirops de maltitol ayant une teneur en maltitol d'environ 52-55 %, comme par exemple le produit de marque LYCASIN® 80/55, ou ceux ayant une teneur en maltitol d'environ 75 %, comme le produit de marque MALTISORB® 75/75.

Les produits de ballast faiblement caloriques sont quant à eux préférentiellement choisis parmi les produits du type polydextrose, notamment les produits commercialisés sous la marque LITESSE®.

Un avantage appréciable des produits selon l'invention est indéniablement leur faible pouvoir cariogène, obtenu grâce à l'utilisation de ces produits édulcorants en lieu et place des sucres traditionnels tels que le saccharose, le glucose ou le fructose. Ceci est particulièrement important quand on considère que ces produits sont destinés à être consommés régulièrement par des enfants et adolescents, éventuellement en dehors des périodes des repas et sans rinçage ultérieur de la cavité buccale. L'utilisation des agents édulcorants précités permet d'autre part l'obtention de produits dont le pouvoir calorique est sensiblement inférieur à celui des produits traditionnels à base de sucres conventionnels.

Les agents édulcorants énumérés ci-dessus sont présents dans la formulation de départ, c'est-à-dire avant gélification et séchage, à raison de 25 % à 78 %, de préférence de 30 % à 75 %, et plus préférentiellement encore de 35 % à 70 %, ces pourcentages étant exprimés en poids de matière commerciale par rapport au poids de la formulation. Les agents édulcorants sont donc généralement présents dans le produit fini en une proportion comprise entre 50 % et 85 %, de préférence entre 60 % et 75 %, et plus préférentiellement encore entre 65 % et 75 %, les pourcentages étant exprimés en poids de matière sèche par rapport à la matière sèche du produit fini.

Comme indiqué précédemment, le sel de calcium est introduit dans la formulation en quantité telle que le produit fini présente une teneur en calcium-élément comprise entre 100 et 500 mg par article unitaire. Le sel de calcium utilisé peut être choisi parmi le carbonate de calcium, le chlorure de calcium, le gluconate de calcium, le glucoheptonate de calcium, le lactate de calcium, le gluconolactate de calcium, le citrate de calcium, le glycérophosphate de calcium, le phosphate de calcium ainsi que leurs mélanges. On peut également utiliser de l'hydroxyapatite microcristalline.

Le carbonate de calcium constitue le sel préféré, d'une part en raison du fait qu'il amène beaucoup de calcium par unité de masse moléculaire, et d'autre part parce qu'il peut être utilisé pour assurer, totalement ou partiellement, l'aération du produit lors de la fabrication, par dégagement de gaz carbonique en présence d'acide.

Le sel de calcium constitue de 10 % à 35 %, de préférence de 10 % à 30 % en poids et plus préférentiellement encore de 12 % à 28 % en poids du produit fini, les pourcentages étant exprimés en sec/sec.

L'agent gélifiant utilisé pour fabriquer le produit conforme à l'invention peut être choisi parmi les gélatines, naturelles ou modifiées, les amidons modifiés, les pectines, la gomme arabique, les carraghénates, les alginates, les gommes de guar et de caroube, l'agar-agar. De préférence, on utilise de la gélatine. Les gélatines d'origine porcine de haut bloom sont préférentiellement choisies. Selon la texture et la densité voulues pour les pâtes gélifiées, on ajuste la quantité de gélatine, sa nature et sa force, et on l'utilise éventuellement en combinaison avec d'autres agents gélifiants.

De façon générale, on introduit dans la formulation de départ une quantité d'agent gélifiant comprise entre 3 % et 15 %, de préférence entre 4 % et 12 % % et plus préférentiellement encore entre 4 % et 10 %, ces pourcentages étant exprimés en poids de matière sèche par rapport à la formulation avant gélification et séchage. Exprimées par rapport à la matière sèche du produit fini, les teneurs en agents gélifiants sont comprises entre 3.5 % et 17 %, de préférence entre 4 % et 13 % et plus préférentiellement encore entre 4.5 % et 10 % en poids pour poids.

La formulation peut également contenir, le cas échéant, des agents moussants comme en particulier l'albumine d'oeuf, ou des agents de battage comme par exemple les produits connus sous l'appellation HYFOAMA® NK ou HYFOAMA® DS, qui vont aider à la formulation de la masse et à sa stabilisation. Ces agents, lorsqu'ils sont présents, le sont en une quantité généralement inférieure à 4 % de la formulation avant gélification et séchage.

La teneur en eau de la pâte gélifiée sans sucre conforme à l'invention se situe généralement entre 10 % et 18 % et de préférence entre 12 % et 17 %. Cette teneur en eau peut être adaptée dans une certaine mesure, en fonction de la texture recherchée pour le produit final. La composition de la venue destinée à être coulée dans les moules d'amidon ou étalée ou extrudée, doit être soigneusement choisie en conséquence. En particulier, quand les produits sont formés par coulage de la venue dans des empreintes formées dans des coffrets d'amidon, la teneur en eau de la formulation doit être ajustée de façon à ce que le sirop ne soit pas trop visqueux, mais également de façon à ce qu'il ne se produise pas de phénomène de croûtage à la surface des produits, ce phénomène étant essentiellement occasionné par une trop forte teneur en eau du mélange et à un pompage trop rapide de l'eau par l'amidon.

Le produit diététique ou pharmaceutique à haute teneur en calcium conforme à l'invention peut également contenir des acides alimentaires, des colorants, des arômes, des édulcorants de synthèse, et des préservateurs ou conservateurs.

Les acides sont généralement choisis parmi l'acide citrique, l'acide lactique, l'acide malique et l'acide tartrique, l'acide citrique étant préférentiellement utilisé. L'acide peut être ajouté dans la formulation dans plusieurs buts, éventuellement cumulatifs : soit abaisser le pH en vue de faciliter la gélification, soit conférer tout simplement un goût acide à l'article fini. De l'acide peut également être saupoudré à la surface de la pâte gélifiée : on préfère alors utiliser de l'acide citrique encapsulé.

Les colorants et les préservateurs ou conservateurs sont choisis parmi les produits communément utilisés en confiserie ou en pharmacie.

Les arômes peuvent être adaptés très facilement à la classe d'âge des consommateurs visés. Ainsi, on utilise des arômes du type fraise pour les enfants et des arômes du type cola pour les adolescents. Des arômes du type banane ou citron vert peuvent être utilisés pour les adultes.

Des édulcorants artificiels, tels que l'aspartame ou l'acésulfame K, peuvent enfin, au besoin, être ajoutés à la formulation.

La pâte gélifiée sans sucre conforme à l'invention peut également subir des traitements de finition tels que huilage-cirage, dragéification dure ou tendre, candissage, ou saupoudrage. On utilise pour ces traitements de finition les mêmes agents sucrants que ceux employés pour la constitution de la pâte gélifiée proprement dite, et plus particulièrement le maltitol et les sirops de maltitol, ainsi que le xylitol, qui peut apporter une sensation de fraîcheur instantanée, ou "cooling effect", lors de l'ingestion du produit.

Comme indiqué précédemment, le produit conforme à l'invention peut également contenir une faible quantité de vitamine D, généralement comprise entre 40 et 1200 unités internationales (U.I.), de préférence entre 80 et 800 U.I. et plus préférentiellement encore entre 250 et 650 U.I., cette quantité étant exprimée par article unitaire. La vitamine D, à la fois vitamine et hormone, agit en effet activement au niveau de l'os. Sous sa forme active, elle favorise l'absorption intestinale du calcium et sa fixation sur l'os ; elle en limite aussi les pertes urinaires. La vitamine D est choisie parmi la vitamine D2, ou ergocalciférol, ou la vitamine D3, ou cholécarciférol, ainsi que leurs mélanges. La vitamine D3 est préférée, et on l'utilise avantageusement sous une forme pulvérulente contenue dans une matrice qui augmente sa stabilité.

Le procédé de préparation de la pâte gélifiée selon l'invention est caractérisé par le fait qu'il comprend les étapes suivantes :
- une étape de mélange des principaux ingrédients de la formulation, à savoir notamment le ou les agents sucrants, le ou les agents gélifiants, le ou les sels de calcium, et éventuellement la vitamine D,
- une étape dans laquelle on opère un moussage de ladite formulation, par aération mécanique et/ou chimique,
- une étape de formage du produit, soit par coulage dans des moules d'amidon, soit par extrusion, soit par dépôt sur table froide et coupage,
- une étape de gélification et de séchage du produit, par maintien du produit formé pendant une période de temps pouvant aller de 6 heures à 10 jours, éventuellement dans une atmosphère ventilée,
- une étape de finition, au cours de laquelle on procède à des opérations telles que notamment démoulage, dépoudrage, lustrage, huilage, cirage, dragéification, candissage ou saupoudrage,
- une étape finale de conditionnement.

Selon une réalisation préférentielle de l'invention, le formage est réalisé par coulage de la venue dans des moules d'amidon comportant des empreintes de dessins et de calibres voulus. De préférence, la fabrication des produits conformes à l'invention se fait alors dans une installation de coulage à amidon du type MOGUL, bien connue de l'homme de l'art.

Dans une telle installation MOGUL, des coffrets sont remplis d'amidon en poudre dans une machine, et l'amidon est ameubli et lissé en même temps, puis des reliefs de plâtre forment des creux dans l'amidon avec la forme voulue des articles. Les coffrets sont ensuite amenés sous des entonnoirs, et une quantité mesurée de la formulation est coulée dans les empreintes qui ont ainsi été réalisées. Les coffrets dans lesquels la formulation a ainsi été coulée sont laissés reposer entre 1 et 10 jours, suivant le poids et la texture voulue pour le produit fabriqué, pour que l'amidon puisse enlever à ces articles l'humidité superflue. Eventuellement, un peu d'amidon peut être rapidement tamisé sur la surface des produits. Dès que les coffrets ont été laissés reposer assez longtemps pour que les articles aient la fermeté désirée, les coffrets sont ramenés soit automatiquement, soit manuellement, vers le MOGUL où le coffret est complètement retourné, de sorte que la poudre d'amidon tombe avec les articles solidifiés. Ces derniers restent sur des tamis où on leur enlève, par brossage et soufflage, la poudre d'amidon qui colle encore et ces articles sont ensuite sortis de l'installation MOGUL par des plans inclinés ou par des dispositifs de transport adéquats. La poudre d'amidon tombe quant à elle à travers les tamis. Elle est séchée puis recyclée.

A la sortie de l'installation MOGUL, certains produits ne subissent plus d'autres traitements et peuvent donc être directement acheminés vers le conditionnement. Lorsque des traitements ultérieurs sont désirés, tels que brillantage, huilage, sucrage, candissage, ou dragéification, ces traitements sont réalisés dans une installation séparée.

Selon une autre réalisation préférentielle de l'invention, on procède à la préparation de la formulation en réalisant dans une première cuve le mélange des agents sucrants, et en réalisant, dans une autre cuve, la dispersion dans l'eau de la gélatine, à une matière sèche d'environ 25 à 45 % et à une température inférieure à 70°C afin de ne pas dénaturer la gélatine, puis on procède au mélange du sirop contenant les agents sucrants et de la dispersion de gélatine sous agitation, à une température de 50°C à 70°C.

Selon un mode de réalisation avantageux du procédé selon l'invention, on ajoute alors directement dans le mélange obtenu le sel de calcium, de préférence constitué par le carbonate de calcium, sous forme de poudre, ou de pâte aqueuse.

Il faut souligner à ce propos que cette démarche va parfaitement à l'encontre de celle qui est généralement suivie par l'homme du métier spécialisé dans la fabrication de ce type de produits. En effet, l'homme du métier en question cherche toujours à obtenir une formulation qui se trouve sous la forme d'une solution ou d'un sirop et il ne conçoit donc que l'utilisation de produits solubles dans l'eau ou celle de présolutions aqueuses ou de solutions alcooliques, au cas où certains des produits destinés à entrer dans la composition se révéleraient insolubles ou peu solubles dans l'eau.

De ce fait, il existait un fort préjugé à l'égard de la possibilité de réaliser une pâte gélifiée contenant des quantités très élevées de sels de calcium, quantités pouvant atteindre jusqu'à 35 % en poids et voisines le plus généralement de 20 % en poids, car les sels de calcium ne sont pas réputés avoir une excellente solubilité dans l'eau. Ainsi, l'homme du métier pouvait redouter de ne pas être à même d'obtenir un sirop homogène pour procéder à la coulée ou de devoir ajouter des quantités d'eau telles que les opérations subséquentes de gélification et de séchage s'en trouveraient fortement compromises (apparition du phénomène de croûtage notamment).

La Société Demanderesse a eu le mérite de montrer qu'en fait il n'était pas nécessaire d'obtenir une dissolution complète du sel de calcium et qu'il était même possible d'ajouter le sel de calcium directement sous forme de poudre dans le sirop destiné à être coulé, tout en obtenant en définitive d'excellentes pâtes gélifiées, très homogènes et très souples, ne laissant apparaître aucune sensation de sableux sous la langue.

L'aération de la pâte avant coulage ou extrusion est réalisée de manière connue en soi, soit par aération mécanique ou chimique, par exemple à l'aide d'injection d'air, ou à l'aide de batteurs à air comprimé.

Il est généralement préféré d'ajouter les additifs tels qu'arômes, colorants, édulcorants de synthèse, et conservateurs au moment ou à la fin de l'étape d'aération, en fonction du type de technique d'aération choisi, juste avant l'étape de coulage.

On dispose ainsi de produits diététiques ou pharmaceutiques à haute teneur en calcium qui se présentent sous une forme et une texture parfaitement acceptées par tous les sujets ou les patients, et qui présentent l'avantage de pouvoir être consommés de manière très régulière, sans réticence, par les enfants et les adolescents notamment.

Les exemples de réalisation qui suivent, donnés à titre purement illustratif, permettent de mieux décrire l'invention.

### EXEMPLE 1 :

62 kg d'un sirop de maltitol commercialisé sous la marque LYCASIN® 80/55 par la Société ROQUETTE FRERES, ayant une matière sèche de 75 %, sont introduits dans une cuve thermostatée maintenue à une température d'environ 70°C.

7 kg de gélatine en poudre (Bloom : 180-250) sont dispersés dans de l'eau, dans une autre cuve, à une température d'environ 60°C, de façon à obtenir une matière sèche de 40 % en poids. La dispersion de gélatine obtenue est laissée en agitation pendant 2 heures de façon à obtenir un bon gonflement et une bonne solubilisation de la gélatine.

Les deux solutions ainsi obtenues sont alors co-introduites, sous agitation, dans une troisième cuve maintenue à une température de 60°C. 19 kg de carbonate de calcium en poudre sont ensuite progressivement introduits dans le mélange maintenu sous agitation et la suspension résultante peut alors être acheminée vers un appareil d'aération du type EUROMATIC, dans lequel l'aération est assurée par injection d'air comprimé.

Un colorant alimentaire et un arôme artificiel sont ajoutés et la venue obtenue, présentant une matière sèche d'environ 76 % et une température de 60°C, est alors coulée dans des moules d'amidon comportant des empreintes permettant d'obtenir un poids d'article unitaire fini d'environ 2.4 grammes.

Environ 0,8 kg de la venue sont coulés par moule d'amidon. La gélification et le séchage sont laissés opérer pendant une durée de 48 heures, après quoi les articles sont démoulés, brossés et dépoussiérés puis lustrés, cirés dans un cylindre conventionnel, à l'aide d'un mélange de cire d'abeille et d'huile végétale hydrogénée.

Les produits obtenus présentent une humidité de 13 %, sont souples et homogènes, et présentent des qualités organoleptiques unanimement appréciées par un panel de dégustation.

### Exemple 2 :

42 kg du sirop de maltitol de marque LYCASIN® 80/55, d'une matière sèche de 75 %, sont introduits dans une cuve thermostatée maintenue à une température d'environ 70°C.

22 kg de sorbitol poudre de marque NEOSORB® commercialisé par la Société ROQUETTE FRERES sont progressivement dissous dans le sirop de LYCASIN®, sous agitation, avec addition de 6 litres d'eau.

6 kg de gélatine de Bloom 180-250 sont dispersés, à une matière sèche de 40 %, dans une autre cuve maintenue à une température de 55°C.

Après gonflement et solubilisation de la gélatine, les deux préparations sont mélangées dans une autre cuve et 15 kg de carbonate de calcium sont alors introduits sous agitation.

Après aération sur un appareil du type EUROMATIC, un colorant jaune alimentaire et un arôme banane sont ajoutés et le mélange obtenu, présentant une matière sèche de 75 % et maintenu à une température de 60°C, est coulé dans des moules d'amidon comportant des empreintes calibrées de manière à obtenir un poids d'article fini d'environ 4.8 g.

La gélification et le séchage sont terminés après une durée de 76 heures en atmosphère ambiante, après quoi les articles sont démoulés, brossés et dépoussiérés.

## Revendications

1. Produit diététique ou pharmaceutique à haute teneur en calcium, **caractérisé en ce qu'**il se présente sous la forme d'une pâte gélifiée sans sucre contenant de 10 à 35 %, de préférence de 10 à 30 %, de sel de calcium et de 3,5 à 17 % d'agents gélifiants, les pourcentages étant exprimés en poids de la matière sèche par rapport à la matière sèche du produit fini, et **en ce qu'**il contient de 100 à 500 mg de calcium par article unitaire.

2. Produit diététique ou pharmaceutique selon la revendication 1, **caractérisé par le fait que** le calcium est présent par l'intermédiaire d'un sel choisi parmi le carbonate de calcium, le chlorure de calcium, le gluconate de calcium, le glucoheptonate de calcium, le lactate de calcium, le gluconolactate de calcium, le citrate de calcium, le glycérophosphate de calcium, le phosphate de calcium et/ou l'hydroxyapatite microcristalline, le carbonate de calcium étant préféré.

3. Produit diététique ou pharmaceutique selon l'une ou l'autre des revendications 1 et 2, **caractérisé par le fait que** le sel de calcium est présent en une quantité de 12 à 28 % en poids du produit fini.

4. Produit diététique ou pharmaceutique selon l'une quelconque des revendications 1 à 3, **caractérisé par le fait qu'**il contient, en tant qu'agents édulcorants, des polyols, notamment le sorbitol, le maltitol, le mannitol, l'érythritol, le xylitol, l'isomalt, les sirops de sorbitol et les sirops de maltitol, et/ou des produits de ballast faiblement caloriques tels que les polyglucoses et les polyfructoses.

5. Produit diététique ou pharmaceutique selon la revendication 4, **caractérisé par le fait que** les agents édulcorants sont présents dans le produit fini dans une proportion de 50 à 85 %, de préférence de 60 à 75 %, et plus préférentiellement encore de 65 à 75 %, les pourcentages étant exprimés en poids de matière sèche par rapport à la matière sèche du produit fini.

6. Produit diététique ou pharmaceutique selon l'une quelconque des revendications 1 à 5, **caractérisé par le fait que** les agents gélifiants sont choisis parmi les gélatines, les amidons modifiés, les pectines, la gomme arabique, les carraghénates, les alginates, les gommes de guar et de caroube, l'agar-agar, les gélatines étant particulièrement préférées.

7. Produit diététique ou pharmaceutique selon l'une quelconque des revendications 1 à 6, **caractérisé par le fait qu'**il contient de 4 à 13 %, et de préférence de 4.5 à 10 % d'agents gélifiants.

8. Produit diététique ou pharmaceutique selon l'une quelconque des revendications 1 à 7, **caractérisé par le fait qu'**il contient également des agents moussants ou des agents de battage, des acides alimentaires, des colorants, des arômes, des conservateurs ou des préservateurs, et des agents édulcorants de synthèse.

9. Produit diététique ou pharmaceutique selon l'une quelconque des revendications 1 à 8, **caractérisé par le fait qu'**il présente un poids unitaire compris entre 0.5 et 6 g, de préférence entre 1.5 et 5.5 g, et plus préférentiellement encore entre 1.8 et 5.2 g, les articles destinés aux enfants ayant de préférence un poids unitaire d'environ 2 à 3 g et les articles destinés aux adolescents et aux adultes ayant de préférence un poids unitaire d'environ 3 à 5 g.

10. Produit diététique ou pharmaceutique selon l'une quelconque des revendications 1 à 9, **caractérisé par le fait qu'**il présente une teneur en eau de 10 à 18 %, de préférence de 12 à 17 %.

11. Produit pharmaceutique selon l'une quelconque des revendications 1 à 10, **caractérisé par le fait qu'**il contient de 40 à 1200 U.I., de préférence de 80 à 800 U.I. et plus préférentiellement encore de 250 à 650 U.I. de vitamine D, cette quantité étant exprimée par article unitaire.

12. Procédé de préparation du produit diététique ou pharmaceutique selon l'une quelconque des revendications 1 à 11, **caractérisé par le fait qu'**il comprend les étapes suivantes :
- une étape de mélange des principaux ingrédients de la formulation, notamment le ou les polyols, le ou les agents gélifiants, le ou les sels de calcium, et éventuellement la vitamine D,
- une étape dans laquelle on opère un moussage de ladite formulation, par aération mécanique et/ou chimique,
- une étape de formage du produit,
- une étape de gélification et de séchage du produit, par maintien du produit formé pendant une période de temps de 6 heures à 10 jours,
- une étape de finition, dans laquelle on procède à des opérations telles que notamment démoulage, dépoudrage, lustrage, huilage, cirage, dragéification, candissage ou saupoudrage,
- une étape de conditionnement.

13. Procédé selon la revendication 12, **caractérisé par le fait que** l'étape de formage est réalisée par coulage dans des moules d'amidon.

14. Procédé selon l'une ou l'autre des revendications 12 et 13, **caractérisé par le fait que** le ou les sels de calcium sont ajoutés directement sous forme de poudre.

## Patentansprüche

1. Diätetisches oder pharmazeutisches Produkt mit hohem Calcium-Gehalt, **dadurch gekennzeichnet, dass** es in Form einer gelierten Paste ohne Zucker vorliegt, enthaltend 10 bis 35 und vorzugsweise 10 bis 30% Calciumsalz und 3,5 bis 17% Geliermittel, wobei die Prozentsätze als Gewicht der Trockenmasse, bezogen auf die Trockenmasse des Endprodukts, angegeben sind, und dass es 100 bis 500 mg Calcium pro Einheitsartikel enthält.

2. Diätetisches oder pharmazeutisches Produkt gemäß Anspruch 1, **dadurch gekennzeichnet, dass** das Calcium als Salz vorliegt, welches unter Calciumcarbonat, Calciumchlorid, Calciumgluconat, Calciumglucoheptonat, Calciumlactat, Calciumgluconolactat, Calciumzitrat, Calciumglycerophosphat, Calciumphosphat und/oder unter mikrokristallinem Hydroxyapatit ausgewählt ist, wobei Calciumcarbonat bevorzugt wird.

3. Diätetisches oder pharmazeutisches Produkt gemäß Anspruch 1 oder 2, **dadurch gekennzeichnet, dass** das Calciumsalz in einer Menge von 12 bis 28 Gew.% des Endprodukts vorhanden ist.

4. Diätetisches oder pharmazeutisches Produkt gemäß einem der Ansprüche 1 bis 3, **dadurch gekennzeichnet, dass** es, als Süßungsmittel, Polyole, insbesondere Sorbit, Maltit, Mannit, Erythrit, Xylit, Isomalt, Sirup-Produkte aus Sorbit und Maltit und/oder schwach kalorische Ballastprodukte wie Polyglucosen und Polyfructosen enthält.

5. Diätetisches oder pharmazeutisches Produkt gemäß Anspruch 4, **dadurch gekennzeichnet, dass** die Süßungsmittel im Endprodukt in einem Mengenanteil von 50 bis 85, vorzugsweise von 60 bis 75 und besonders bevorzugt von 65 bis 75% vorhanden sind, wobei die Prozentsätze als Gewicht der Trockenmasse, bezogen auf die Trockenmasse des Endprodukts, angegeben sind.

6. Diätetisches oder pharmazeutisches Produkt gemäß einem der Ansprüche 1 bis 5, **dadurch gekennzeichnet, dass** die Geliermittel aus Gelatinen, modifizierten Stärken, Pektinen, Gummi arabicum, Karraghenaten, Alginaten, Guar- und Johannisbrot-Gummiprodukten und aus Agar-Agar ausgewählt sind, wobei die Gelatinen besonders bevorzugt sind.

7. Diätetisches oder pharmazeutisches Produkt gemäß einem der Ansprüche 1 bis 6, **dadurch gekennzeichnet, dass** es 4 bis 13 und vorzugsweise 4,5 bis 10% Geliermittel enthält.

8. Diätetisches oder pharmazeutisches Produkt gemäß einem der Ansprüche 1 bis 7, **dadurch gekennzeichnet, dass** es auch synthetische Schäumungs- oder Schlagemittel, Nahrungsmittelsäuren, Farbstoffe, Aromastoffe, Konservierungs- oder Schutzstoffe und Süßungsmittel enthält.

9. Diätetisches oder pharmazeutisches Produkt gemäß einem der Ansprüche 1 bis 8, **dadurch gekennzeichnet, dass** es ein Einheitsgewicht von 0,5 bis 6, vorzugsweise von 1,5 bis 5,5 und besonders bevorzugt von 1,8 bis 5,2 g aufweist, wobei die für Kinder bestimmten Artikel vorzugsweise ein Einheitsgewicht von ca. 2 bis 3 g und die für Jugendliche und Erwachsene bestimmten Artikel vorzugsweise ein Einheitsgewicht von ca. 3 bis 5 g aufweisen.

10. Diätetisches oder-pharmazeutisches Produkt gemäß einem der Ansprüche 1 bis 9, **dadurch gekennzeichnet, dass** es einen Wassergehalt von 10 bis 18 und vorzugsweise von 12 bis 17% aufweist.

11. Pharmazeutisches Produkt gemäß einem der Ansprüche 1 bis 10, **dadurch gekennzeichnet, dass** es 40 bis 1200, vorzugsweise 80 bis 800 und besonders bevorzugt 250 bis 650 IE Vitamin D enthält, wobei diese Menge pro Einheitsartikel angegeben ist.

12. Verfahren zur Herstellung des diätetischen oder pharmazeutischen Produkts gemäß einem der Ansprüche 1 bis 11, **dadurch gekennzeichnet, dass** es die folgenden Stufen umfaßt:
- eine Stufe zur Vermischung der Hauptbestandteile der Formulierung, insbesondere des oder der Polyole, des oder der Geliermittel und des oder der Calciumsalze, sowie gegebenenfalls des Vitamins D,
- eine Stufe, wobei man die genannte Formulierung durch mechanische und/oder chemische Belüftung verschäumt;
- eine Stufe zur Bildung des Produkts,
- eine Stufe zur Gelierung und Trocknung des Produkts, wobei man das gebildete Produkt während einer Zeitdauer von 6 h bis 10 Tagen stehen lässt,
- eine Stufe zur Endfertigung, wobei man Vorgänge wie insbesondere eine Entformung, Entstaubung, Glanzbildung, Behandlung mit Öl, Behandlung mit Wachs, Bildung von Dragees, Bildung von Kandis oder eine Streuung durchführt;
- eine Stufe zur Konditionierung.

13. Verfahren gemäß Anspruch 12, **dadurch gekennzeichnet, dass** die Bildungsstufe durch Gießen in Formen aus Stärke durchgeführt wird.

14. Verfahren gemäß Anspruch 12 oder 13, **dadurch gekennzeichnet, dass** das oder die Calciumsalze direkt in Pulverform zugegeben werden.

## Claims

1. Dietetic or pharmaceutical product with a high calcium content, **characterised in that** it is presented in the form of a sugar-free gelled paste containing from 10 to 35 %, preferably from 10 to 30 %, of calcium salt and from 3.5 to 17 % of gelling agents, the percentages being expressed as weight of dry material relative to the dry material of the finished product, and **in that** it contains from 100 to 500 mg of calcium per unit item.

2. Dietetic or pharmaceutical product according to Claim 1, **characterised in that** the calcium is present via a salt chosen from among calcium carbonate, calcium chloride, calcium gluconate, calcium glucoheptonate, calcium lactate, calcium gluconolactate, calcium citrate, calcium glycerophosphate, calcium phosphate and/or microcrystalline hydroxyapatite, calcium carbonate being preferred.

3. Dietetic or pharmaceutical product according to one or other of the Claims 1 and 2, **characterised in that** the calcium salt is present in a quantity of 12 to 28 % by weight of the finished product.

4. Dietetic or pharmaceutical product according to any one of the Claims 1 to 3, **characterised in that** it contains polyols as sweetening agents, in particular sorbitol, maltitol, mannitol, erythritol, xylitol, isomalt, sorbitol syrups and maltitol syrups, and/or low-calorie filler products such as polyglucoses and polyfructoses.

5. Dietetic or pharmaceutical product according to Claim 4, **characterised in that** the sweetening agents are present in the finished product in a proportion of 50 to 85 %, preferably from 60 to 75 %, and even more preferably from 65 to 75 %, the percentages being expressed as weight of dry material relative to the dry material of the finished product.

6. Dietetic or pharmaceutical product according to any one of the Claims 1 to 5, **characterised in that** the gelling agents are chosen from among the gelatines, modified starches, pectins, gum Arabic, carrageenates, alginates, guar and carob gums and agar-agar, the gelatines being especially preferred.

7. Dietetic or pharmaceutical product according to any one of the Claims 1 to 6, **characterised in that** it contains from 4 to 13 %, preferably from 4.5 to 10 % of gelling agents.

8. Dietetic or pharmaceutical product according to any one of the Claims 1 to 7, **characterised in that** it also contains foaming agents or whipping agents, food acids, colouring agents, flavourings, conserving agents or preservatives and synthetic sweeteners.

9. Dietetic or pharmaceutical product according to any one of the Claims 1 to 8, **characterised in that** it has a unit weight lying between 0.5 and 6 g, preferably between 1.5 and 5.5 g, and even more preferably between 1.8 and 5.2 g, the items intended for children preferably having a unit weight of about 2 to 3 g and the items intended for adolescents and adults preferably having a unit weight of about 3 to 5 g.

10. Dietetic or pharmaceutical product according to any one of the Claims 1 to 9, **characterised in that** it has a water content of 10 to 18 %, preferably from 12 to 17 %.

11. Dietetic or pharmaceutical product according to any one of the Claims 1 to 10, **characterised in that** it contains from 40 to 1200 I.U., preferably from 80 to 800 I.U. and even more preferably from 250 to 650 I.U. of vitamin D, this quantity being expressed per unit item.

12. Process for preparing the dietetic or pharmaceutical product according to any one of the Claims 1 to 11, **characterised in that** it comprises the following stages:
- a stage of mixing the main ingredients of the formulation, namely the polyol(s), the gelling agent(s), the calcium salt(s) and optionally the vitamin D,
- a stage in which the said formulation is converted into a mousse by mechanical and/or chemical aeration,
- a stage in which the product is shaped/moulded,
- a stage in which the product is gelled and dried, by keeping the shaped/moulded product for a period of 6 hours to 10 hours,
- a finishing stage in which operations such as unmoulding, de-dusting, polishing, oiling, waxing, sugar-coating, glazing or dredging are performed,
- a packaging stage.

13. Process according to Claim 12, **characterised in that** the shaping stage is performed by pouring into starch moulds.

14. Process according to one or other of the Claims 12 and 13, **characterised in that** the calcium salt(s) is/are added directly in powder form.
